# FASCICULE DE BREVET EUROPEEN

(11) **EP 0 881 158 B1**
(45) Date de publication et mention de la délivrance du brevet: **17.03.2004**
(21) Numéro de dépôt: 98400777.3
(22) Date de dépôt: 02.04.1998
(51) Int. Cl.: B65D 47/42, A61J 1/14

(54) **Dispositif pour la distribution et le conditionnement de produits liquides stériles**
Vorrichtung zum Spenden und Aufbewahren von sterilen Flüssigkeiten
Device for dispensing and storing sterile fluids

(30) Priorité: 02.04.1997 FR 9703984
(43) Date de publication de la demande: 02.12.1998
(73) Titulaire: REXAM DISPENSING SYSTEMS, 76470 Le Tréport (FR)
(72) Inventeur: Bougamont, Jean-Louis, 76260 Eu (FR); Hennemann, Pascal, 76260 Eu (FR)
(74) Mandataire: Busnel, Jean-Benoît

(56) Documents cités:
- EP-A- 0 332 480
- EP-A- 0 459 498
- EP-A- 0 602 019
- FR-A- 823 360
- FR-A- 2 340 870
- FR-A- 2 422 569
- FR-A- 2 478 589
- US-A- 3 149 758
- US-A- 4 463 880
- US-A- 5 105 993

## Description

La présente invention concerne un dispositif pour la distribution et le conditionnement de produits liquides stériles et plus particulièrement de produits pharmaceutiques à usage ophtalmique.

Il existe déjà des dispositifs de ce type tels que celui qui a fait l'objet de la demande de brevet FR 96 00120.

Ces dispositifs comprennent notamment un réservoir de produit surmonté d'un embout qui est pourvu d'un conduit interne communiquant à son extrémité amont avec le réservoir et débouchant à son extrémité aval à l'extérieur via un orifice d'évacuation. Le conduit interne est généralement obturé de manière étanche, d'une part, au niveau de l'orifice d'évacuation, par un clapet et, d'autre part, au moyen d'une lèvre interne d'étanchéité qui est élastiquement déformable.

Cependant, ces moyens d'obturation, qui sont mobiles ou déformables sont complexes et tant leur fabrication que leur montage sur le dispositif, sont des opérations délicates et onéreuses.

En outre, ces dispositifs ne permettent pas de garantir un mode de distribution au goutte-à-goutte, ce qui constitue un handicap majeur vis-à-vis de produits dont la posologie peut être prescrite de façon stricte.

En outre, parmi ces dispositifs certains fonctionnent sans reprise d'air, ce qui entraîne une déformation du réservoir et nécessite une structure souple. Enfin, d'autres ne peuvent assurer la filtration du flux gazeux aspiré vers le réservoir lors de la reprise d'air consécutive à la délivrance d'une dose de produit. Il en résulte un risque important de contamination ou de pollution du produit.

Le document US 5,105,993 décrit un dispositif de distribution goutte à goutte de produit liquide stérile muni d'un insert poreux permettant l'écoulement du produit tout en arrêtant les contaminants. L'invention propose un perfectionnement de ce dispositif, améliorant le calibrage des gouttes.

Ce but est atteint, selon l'invention, au moyen d'un dispositif pour la distribution et le conditionnement d'un produit liquide stérile du type comprenant un réservoir surmonté d'un embout pourvu d'un conduit interne communiquant à son extrémité amont avec ledit réservoir et débouchant par son extrémité aval à l'extérieur, via un orifice d'évacuation, ledit embout comportant un insert de porosité sélective obstruant au moins partiellement le conduit interne et permettant, à la fois, un écoulement dosé du produit vers l'extérieur et une filtration de l'air aspiré vers l'intérieur en arrêtant les contaminants et/ou polluants biologiques, caractérisé en ce que ledit insert comporte un doigt qui est inséré dans l'orifice d'évacuation et dont l'extrémité fait saillie à l'extérieur, et d'autre part, un capot coiffant ledit embout et portant un élément d'obturation étanche dudit orifice d'évacuation.

Selon un mode de réalisation particulier, ledit élément d'obturation est constitué d'une lèvre périphérique interne destinée à venir en appui par l'extérieur autour de l'orifice d'évacuation, lors de la mise en place du capot sur l'embout.

Selon une variante de réalisation, ledit capot comporte un organe de verrouillage coopérant par encliquetage avec un organe de retenue ménagé sur l'embout.

Selon une autre variante, ledit capot comporte des éléments raidisseurs prenant appui sur l'embout.

Selon une caractéristique avantageuse, ledit insert a un profil adapté pour se loger dans l'extrémité aval dudit conduit.

Selon encore une autre caractéristique, le dispositif est réalisé au moins partiellement avec un matériau contenant un agent chimique bactéricide et/ou antiseptique.

Selon d'autres caractéristiques, la porosité dudit insert est comprise entre 40 % et 60 %, et de préférence, le diamètre des pores est compris entre 5 et 10 µm.

Le cas échéant, la porosité de l'insert est non uniforme et délimite dans sa masse des canaux pour l'écoulement préférentiel du produit.

Le dispositif de la présente invention assure à la fois un dosage précis et reproductible et une protection bactérienne du produit en utilisant des moyens techniques simples.

L'étanchéité du dispositif est complétée et renforcée par l'action du capot.

L'insert poreux est, en outre, un constituant simple, fiable et économique qui a une action synergique avec les autres constituants du dispositif pour assurer conjointement, la régulation du débit de produit, la filtration de l'air aspiré et la nécessaire étanchéité du dispositif.

La présente invention sera mieux comprise à la lecture de la description qui va suivre accompagnée du dessin sur lequel :

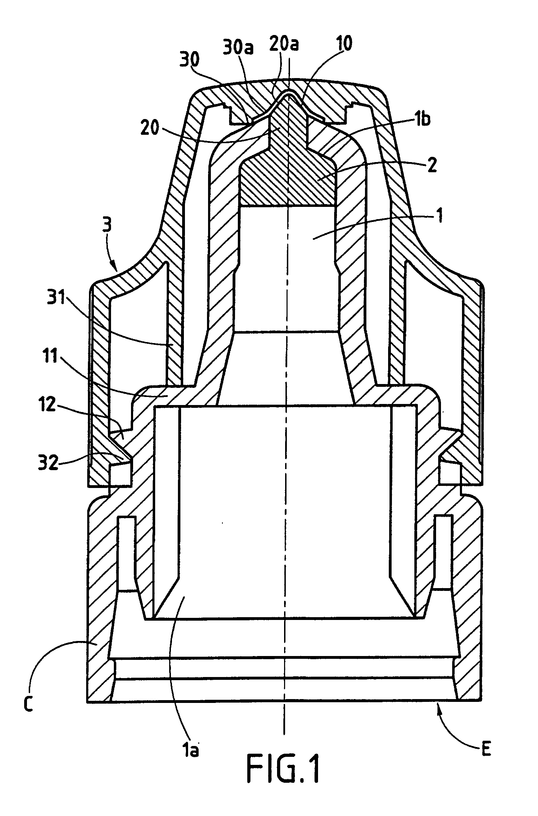

- la figure 1 représente une vue en coupe d'un premier mode de réalisation du dispositif de l'invention.

Le dispositif représenté sur la figure 1est plus particulièrement destiné à la distribution et au conditionnement d'un produit liquide stérile.

Ce dispositif comprend un réservoir (non représenté) surmonté d'un embout E.

L'embout E est pourvu d'une collerette C de raccordement au réservoir et d'un conduit interne 1 communiquant à son extrémité amont 1a avec le réservoir et débouchant, par son extrémité aval 1b, à l'extérieur via un orifice d'évacuation 10.

Le conduit 1 est, au moins partiellement, obstrué par un insert 2 réalisé avec un matériau de porosité sélective.

Plus précisément, cette porosité est telle que, l'insert 2 assure, d'une part, un dosage du produit en ralentissant son débit d'écoulement vers l'extérieur, lors de la mise en pression du réservoir, et , d'autre part, une filtration de l'air aspiré vers l'intérieur lors du retour de la pression interne du réservoir à la pression atmosphérique.

La reprise d'air s'effectue ainsi en arrêtant les contaminants et/ou polluants biologiques.

Sur la figure 1, l'insert 2 a un profil adapté pour se loger dans l'extrémité aval 1b du conduit 1 avec un léger serrage radial ou une faible compression, en occupant toute la section libre.

Par suite, le flux total de produit liquide est amené à traverser l'insert 2, ce qui assure une régulation du débit. Le transfert s'effectue sous pression, d'abord par imprégnation de l'insert, au travers des pores, puis par libération du produit.

La porosité de l'insert 2 est, de préférence, comprise entre 40% et 60 % avec un diamètre de pores compris entre 5 et 10 µm. Ce qui permet de produire, à la fois, un dosage au goutte-à-goutte dans un sens, et un arrêt ou un piégeage des principaux agents contaminants dans l'autre sens.

Le cas échéant, l'insert est imprégné ou réalisé au moins partiellement avec une matière ayant une activité bactéricide (par exemple une substance oligodynamique à base d'argent).

Dans le mode de réalisation de la figure 1, l'insert 2 comporte un doigt 20 qui est inséré dans l'orifice d'évacuation 10 avec un léger serrage radial. L'extrémité 20a du doigt 20 fait saillie à l'extérieur avec un profil en ogive favorisant la formation des gouttes de produit et permettant de calibrer le volume des gouttes de préférence entre 25µl et 50µl.

Le dispositif de l'invention comporte, en outre, un capot 3 destiné à coiffer au moins l'extrémité de l'embout E.

Le capot 3 porte un élément d'obturation étanche de l'orifice d'évacuation 10.

Cet élément est constitué, dans le mode de réalisation de la figure 1, d'une lèvre périphérique 30 réalisée sur la paroi interne du capot 3 et qui est destinée à venir en appui par l'extérieur autour de l'orifice 10 lors de la mise en place du capot 3 sur l'embout E.

La lèvre 30 est relativement rigide et borde une cavité 30a, ménagée sur la paroi interne du capot 3, dans laquelle vient se loger, l'extrémité 20a en ogive du doigt 20 de l'insert 2.

Dans le cas où l'extrémité 20a du doigt 20 est ajustée avec un léger jeu aux dimensions de la cavité 30a, les surfaces mutuelles en regard délimitent un espace mort dans lequel est assurée une protection anti-bactérienne.

Le capot 3 comporte aussi un organe de verrouillage 32 coopérant par encliquetage avec un organe de retenue 12 ménagé sur l'embout E.

La position et la géométrie des organes de verrouillage 32 et de retenue 12 sont déterminées de façon à assurer une mise en appui étanche de la lèvre 30 autour de l'orifice 10 et, donc, un assemblage hermétique du capot 3 sur l'embout E.

Le capot 3 comporte, en outre, des éléments raidisseurs 31 sous forme d'ailettes ou d'un cylindre prenant appui sur un épaulement 11 de l'embout E.

En vue de renforcer la protection biologique du produit; il est prévu, le cas échéant, de réaliser le dispositif (embout E et/ou insert 2 et/ou capot 3) avec un matériau plastique contenant un agent chimique bactéricide et/ou antiseptique.

Selon un autre mode de réalisation, non représenté, la porosité est réalisée de façon non uniforme dans l'insert, ce qui délimite dans sa masse, des canaux pour l'écoulement et/ou la circulation préférentiels du produit.

## Revendications

1. Dispositif pour la distribution et le conditionnement d'un produit liquide stérile du type comprenant un réservoir surmonté d'un embout (E) pourvu d'un conduit interne (1) communiquant à son extrémité amont (1a) avec ledit réservoir et débouchant par son extrémité aval (1b) à l'extérieur via un orifice d'évacuation (10), ledit embout comportant un insert (2) de porosité sélective obstruant au moins partiellement le conduit interne (1) et permettant, à la fois, un écoulement dosé du produit vers l'extérieur et une filtration de l'air aspiré vers l'intérieur en arrêtant les contaminants et/ou polluants biologiques, **caractérisé en ce que** ledit insert (2) comporte un doigt (20) qui est inséré dans l'orifice d'évacuation (10) et dont l'extrémité (20a) fait saillie à l'extérieur, et **en ce que** le dispositif comporte un capot (3) coiffant ledit embout (E) et portant un élément d'obturation étanche dudit orifice d'évacuation (10).

2. Dispositif selon la revendication 1, **caractérisé en ce que** ledit élément d'obturation est constitué d'une lèvre périphérique interne (30) destinée à venir en appui par l'extérieur, autour de l'orifice d'évacuation (10) lors de la mise en place du capot (3) sur l'embout (E).

3. Dispositif selon l'une des revendications précédentes, **caractérisé en ce que** ledit capot (3) comporte un organe de verrouillage (32) coopérant par encliquetage avec un organe de retenue (12) ménagé sur l'embout(E).

4. Dispositif selon l'une des revendications précédentes, **caractérisé en ce que** ledit capot (3) comporte des éléments raidisseurs (31) prenant appui sur l'embout (E).

5. Dispositif selon l'une des revendications précédentes, **caractérisé en ce que** ledit insert (2) a un profil adapté pour se loger dans l'extrémité aval (1b) dudit conduit (1).

6. Dispositif selon l'une des revendications précédentes, **caractérisé en ce qu'**il est réalisé au moins partiellement avec un matériau contenant un agent chimique bactéricide et/ou antiseptique.

7. Dispositif selon l'une des revendications précédentes, **caractérisé en ce que** la porosité dudit insert (2) est comprise entre 40 % et 60 %.

8. Dispositif selon l'une des revendications précédentes, **caractérisé en ce que** le diamètre des pores dudit insert (2) est compris entre 5 et 10 µm.

9. Dispositif selon l'une des revendications précédentes, **caractérisé en ce que** la porosité de l'insert (2) est non uniforme et délimite dans sa masse des canaux pour l'écoulement préférentiel dudit produit.

## Claims

1. A device for packaging and dispensing a sterile liquid product, of the type comprising a reservoir on which is mounted a nozzle (E) provided with an inner conduit (1) communicating with said reservoir at its upstream end (1a) and opening to the outside at its downstream end (1b) via an evacuation orifice,
said nozzle comprising an insert (2) of selective porosity obstructing the inner conduit (1) at least partially and allowing both a metered flow of the product towards the outside and a filtration of the air sucked towards the inside, stopping biologically polluting and/or contaminating agents, **characterized in that** said insert (2) comprises a finger (20) which is inserted in the evacuation orifice (10), whose end (20a) projects to the outside, and **in that** the device comprises a cap (3) covering said nozzle (E) and bearing an element for hermetically obturating said evacuation orifice (10).

2. The device of Claim 1, **characterized in that** said obturation element is constituted by an inner peripheral lip (30) adapted to abut around the evacuation orifice (10) via the outside, when the cap (30) is placed on the nozzle (E).

3. The device according to any one of the preceding claims, **characterized in that** said cap (3) comprises a locking member (32) cooperating by clipping with a retaining member (12) arranged on the nozzle (E).

4. The device according to any one of the preceding claims, **characterized in that** said cap (3) comprises stiffening elements (31) abutting on the nozzle (E).

5. The device according to any one of the preceding claims, **characterized in that** said insert (2) presents a profile adapted to be housed in the downstream end (1b) of said conduit (1).

6. The device according to any one of the preceding claims, **characterized in that** it is made at least partially of a material containing a bactericidal and/or antiseptic chemical agent.

7. The device according to any one of the preceding claims, **characterized in that** the porosity of said insert (2) is included between 40% and 60%.

8. The device according to any one of the preceding claims, **characterized in that** the diameter of the pores of said insert (2) is included between 5 and 10 µm.

9. The device according to any one of the preceding claims, **characterized in that** the porosity of the insert (2) is non-uniform and defines in its mass channels for the preferential flow of the product.

## Patentansprüche

1. Vorrichtung zum Spenden und Aufbewahren einer sterilen Flüssigkeit, von der Art, dass sie einen Behälter umfasst, auf den ein Ansatzstück (E) aufgesetzt ist, das mit einer internen Leitung (1) versehen ist, die an ihrem oberen Ende (1a) mit dem besagten Behälter in Verbindung steht und mit ihrem unteren Ende (1b) durch eine Abflussöffnung (10) nach Außen mündet, wobei das besagte Ansatzstück einen Einsatz (2) mit selektiver Porosität umfasst, der wenigstens zum Teil die interne Leitung (1) blockiert und zugleich ein dosiertes Ausfließen des Produkts nach Außen und ein Filtrieren der nach Innen eingesaugten Luft erlaubt, wobei die biologischen kontaminierenden undloder verschmutzenden Stoffe zurückgehalten werden,
**dadurch gekennzeichnet,**
**dass** der besagte Einsatz (2) einen Finger (20) umfasst, der in die Abflussöffnung (10) eingefügt ist und dessen Ende (20a) nach Außen einen Vorsprung bildet, und dass die Vorrichtung eine Kappe (3) umfasst, die das besagte Ansatzstück (E) bedeckt und ein Element zum dichten Verschluss der besagten Abflussöffnung (10) trägt.

2. Vorrichtung nach Anspruch 1,
**dadurch gekennzeichnet,**
**dass** das besagte Element zum Verschluss aus einer internen Peripherielippe (30) gebildet ist, die dazu vorgesehen ist, von Außen um die Abflussöffnung (10) herum in Anlage zu kommen, wenn die Kappe (3) auf dem Ansatzstück (E) angebracht ist.

3. Vorrichtung nach einem der vorhergehenden Ansprüche,
**dadurch gekennzeichnet,**
**dass** die besagte Kappe (3) ein Verriegelungsorgan (32) umfasst, das durch Aufschnappen mit einem auf dem Ansatzstück (E) angebrachten Haltorgan (12) zusammenwirkt.

4. Vorrichtung nach einem der vorhergehenden Ansprüche,
**dadurch gekennzeichnet,**
**dass** die besagte Kappe (3) Spannelemente (31) umfasst, die auf dem Ansatzstück (E) zur Anlage kommen.

5. Vorrichtung nach einem der vorhergehenden Ansprüche,
**dadurch gekennzeichnet,**
**dass** der besagte Einsatz (2) ein passendes Profil besitzt, um sich in dem oberen Ende (1b) der besagten Leitung (1) einzufügen.

6. Vorrichtung nach einem der vorhergehenden Ansprüche,
**dadurch gekennzeichnet,**
**dass** sie wenigstens zum Teil aus einem Material gebildet ist, das einen bakteriziden und/oder antiseptischen chemischen Wirkstoff enthält.

7. Vorrichtung nach einem der vorhergehenden Ansprüche,
**dadurch gekennzeichnet,**
**dass** die Porosität des besagten Einsatzes (2) zwischen 40% und 60% liegt.

8. Vorrichtung nach einem der vorhergehenden Ansprüche,
**dadurch gekennzeichnet,**
**dass** der Durchmesser der Poren des besagten Einsatzes (2) zwischen 5 und 10 µm liegt.

9. Vorrichtung nach einem der vorhergehenden Ansprüche,
**dadurch gekennzeichnet,**
**dass** die Porosität des Einsatzes (2) nicht uniform und festgelegt in der Menge der Kanäle zum bevorzugten Ausfließen des besagten Produkts ist.
